# EUROPEAN PATENT APPLICATION

(11) **EP 3 892 615 A1**
(43) Date of publication of application: **13.10.2021**
(21) Application number: 20315137.8
(22) Date of filing: 09.04.2020
(51) Int. Cl.: C07D 209/08

(54) **PROCESS FOR THE PREPARATION OF SILODOSIN**

(71) Applicant: Minakem, 59640 Dunkerque (FR)
(72) Inventor: Baran, Phil, San Diego, CA 92130 (US); Vantourout, Julien, San Diego, CA 92122 (US); Chen, Tie-Gen, Zhongshan, 528400 (CN); Delbrayelle, Dominique, 59500 Douai (FR); Echeverria, Pierre-Georges, 59800 Lille (FR)
(74) Representative: Plasseraud IP

(57) **Abstract**

The present invention relates to a new process of preparation of Silodosin, or a pharmaceutically acceptable salt thereof. Another aspect of the invention concerns compounds of formula II: 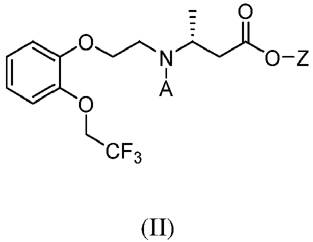 or their pharmaceutically acceptable salts thereof, and their use for the preparation of Silodosin or a pharmaceutically acceptable salt thereof.

## Description

The present invention relates to a new process of preparation of Silodosin, or a pharmaceutically acceptable salt thereof. Another aspect of the invention concerns a compound of formula II: 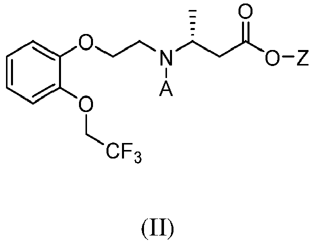 or their pharmaceutically acceptable salts thereof, and their use for the preparation of Silodosin or a pharmaceutically acceptable thereof.

### BACKGROUND OF THE INVENTION

Silodosin is an indoline compound chemically known as 1-(3-hydroxypropyl)-5-[(2*R*)-2-[2-[2-(2,2,2-trifluoroethoxy)phenoxy]ethylamino]propyl]-2,3-dihydroindole-7-carboxamide and represented by formula I:

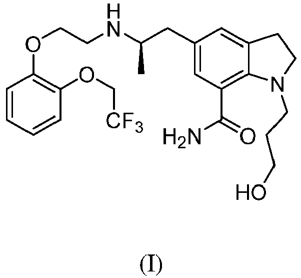

Sidolosin was first disclosed in US 5,387,603 as well as its synthesis and its use as a therapeutic agent for the treatment of lower uninary tract symptoms and dysuria associated with benign prostatic hyperplasia.

Since then, many other synthetic routes have been disclosed.

Historically, approaches towards Silodosin rely on the reaction between a fragment *i* and a fragment *ii*:

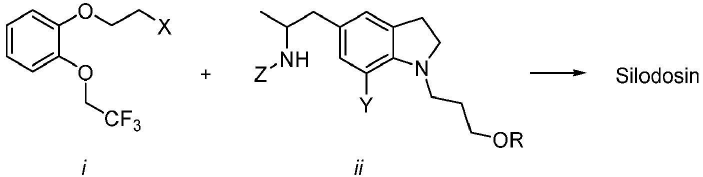

Calogero et al. (Eur. J. Org. Chem., 2015, 6011-6016) discloses the preparation of a fragment *ii* based on Cu^{I}-catalysed C-C arylation of indoline, regioselective cyanation and diasteroselective reductive amination. The fragment *ii* is then reacted with the fragment *i.*

WO 2012/062229 A1 discloses the preparation of a fragment *ii* wherein Z is a phenylethyl group, followed by the reaction with the fragment *i* and deprotection of the amino group.

EP 2 768 806 B1 discloses the preparation of a fragment *ii* as a racemic mixture which is then subjected to an enantiomeric procedure and then reacted with the fragment *i*.

WO 2014/118606 A2 discloses the preparation of a fragment *ii* wherein Z is a *tert-*butylsulfinyl group. The sulfinyl group is then removed to give an enantiomerically pure fragment which is then reacted with reacted with the fragment *i*.

However, these methods require a long synthetic route that is not efficient for industrial processes. Indeed, these methods provide Silodosin with low yields and/or require a step of separation of enantiomers.

Therefore, there is still a need for a process of preparation of Silodosin that requires fewer steps and that is more efficient and convergent, while being inherently safe and scalable.

### SUMMARY OF THE INVENTION

The inventors have now succeeded in developing a novel process for the preparation of Silodosin in good yield that is viable at an industrially relevant scale.

The present invention therefore relates to a process for the preparation of Silodosin of formula I, 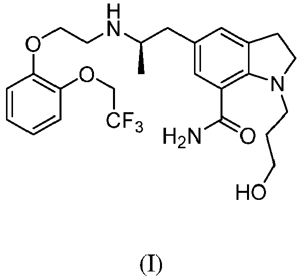 or a pharmaceutically acceptable salt or solvate thereof, comprising the following steps in the following order:
a) preparing a compound of formula II: 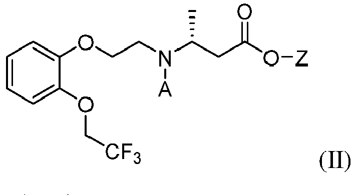 wherein
   **A** is an amine protecting group, and
   **Z** is selected from H, Na, 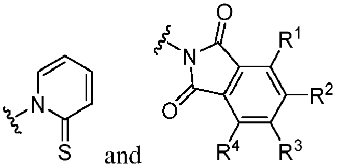 wherein **R¹**, **R²**, **R³** and
   **R⁴** are independently H or halo;
b) reacting said compound of formula II with a compound of formula III: 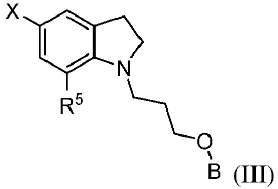 wherein
   **X** is selected from halogen, C1-C6-alkoxy, C1-C6-alkylsulfonyloxy, halo-C1-C6-alkylsulfonyloxy, di-C1-C6-alkylaminosulfonyloxy and di-C1-C6-alkylcarbamoyloxy,
   **R⁵** is CN or C(O)NH₂, and
   **B** is an hydroxyl protecting group;
c) cleaving the groups **A** and **B**.

The invention also relates to a compound of formula II: 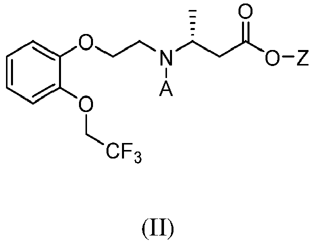 and pharmaceutically acceptable salts thereof,
wherein
**A** is an amine protecting group, and
**Z** is selected from **H**, Na, 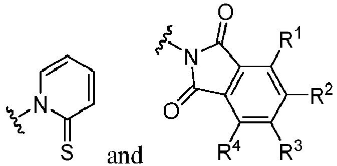 wherein **R¹**, **R²**, **R³** and **R⁴** are independently H or halo.

The invention further relates to the use of the compound of formula II as a synthetic intermediate for the preparation of Silodosin or a pharmaceutically acceptable salt or solvate thereof.

### DETAILED DESCRIPTION OF THE INVENTION

The present invention relates to a process for the preparation of Silodosin of formula I, 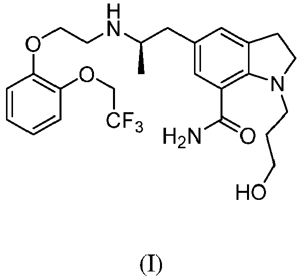 or a pharmaceutically acceptable salt or solvate thereof, comprising the following steps in the following order:
a) preparation of a compound of formula II: 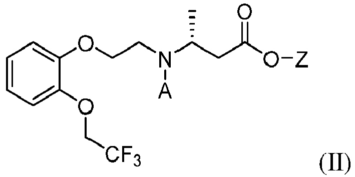 wherein
   **A** is an amine protecting group; preferably **A** is a group selected from C1-C4-alkyloxycarbonyl, aryl-C1-C2-alkyloxycarbonyl, C1-C4-alkylcarbonyl, arylsulfonyl, C1-C4-alkylsulfonyl, halo-C1-C4-alkylsulfonyl, C1-C4-alkyl, C1-C4-alkenyl and aryl-C1-C2-alkyl; more preferably **A** is selected from C1-C4-alkyloxycarbonyl, aryl-C1-C2-alkyloxycarbonyl, C1-C4-alkylcarbonyl and aryl-C1-C2-alkyl; even more preferably, **A** is a group selected from *tert-*butyloxycarbonyl, benzyloxycarbonyl, acetyl and benzyl; still more preferably **A** is *tert*-butyloxycarbonyl; and
   **Z** is selected from H, Na, 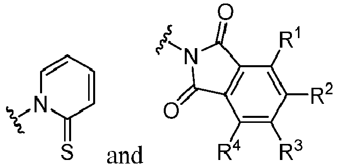 wherein **R¹**, **R²**, **R³** and
   **R⁴** are independently H or halo, preferably **R¹**, **R²**, **R³** and **R⁴** are independently H or chloro, more preferably **R¹, R²**, **R³** and **R⁴** are H; preferably, **Z** is selected from
   H and 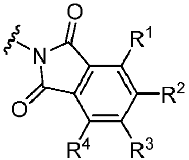 wherein **R¹**, **R²**, **R³** and **R⁴** are independently H or halo, preferably **R¹, R²**, **R³** and R**⁴** are independently H or chloro, more preferably **R¹, R²**, **R³** and **R⁴** are **H**; still more preferably, **Z** is selected from H and 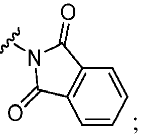
b) reaction of said compound of formula II with a compound of formula III: 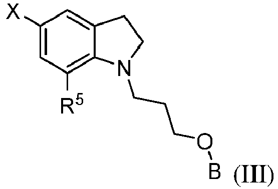 wherein
   **X** is selected from halogen, C1-C6-alkoxy, C1-C6-alkylsulfonyloxy, halo-C 1-C6-alkylsulfonyloxy, di-C1-C6-alkylaminosulfonyloxy and di-C1-C6-alkylcarbamoyloxy, preferably **X** is selected from halogen, C1-C4-alkoxy, C1-C4-alkylsulfonyloxy, halo-C1-C4-alkylsulfonyloxy, di-C1-C4-alkylaminosulfonyloxy and di-C1-C4-alkylcarbamoyloxy, more preferably **X** is selected from iodo, bromo, C1-C2-alkoxy, C1-C2-alkylsulfonyloxy, halo-C1-C2-alkylsulfonyloxy, di-C1-C2-alkylaminosulfonyloxy and di-C1-C2-alkylcarbamoyloxy, still more preferably, **X** is selected from iodo, bromo, methoxy, trifluoromethylsulfonyloxy, dimethylaminosulfonyloxy and dimethylcarbamoyloxy;
   **R⁵** is CN or C(O)NH₂, preferably **R⁵** is CN; and
   **B** is an hydroxyl protecting group, preferably **B** is a group selected from aryl-C1-C6-alkyl, arylcarbonyl, C1-C6-alkylcarbonyl, C1-C6-alkyl, C1-C6-alkenyl, C1-C6-alkyldi-C1-C4-alkylsilyl, C1-C6-alkyldiarylsilyl and tetrahydropyranyl, more preferably **B** is a group selected from aryl-C1-C4-alkyl, arylcarbonyl, C1-C4-alkylcarbonyl, C1-C4-alkyl, C1-C4-alkenyl, C1-C4-alkyldi-C1-C2-alkylsilyl, C1-C4-alkyldiarylsilyl and tetrahydropyranyl, still more preferably **B** is a group selected from aryl-C1-C4-alkyl, arylcarbonyl, C1-C4-alkylcarbonyl and tetrahydropyranyl, even more preferably **B** is a group selected from benzyl, benzoyl, acetyl and tetrahydropyranyl, even more preferably **B** is benzyl;
c) cleaving the groups **A** and **B.**

The step a) of the process of the invention consists in preparing a compound of formula II: 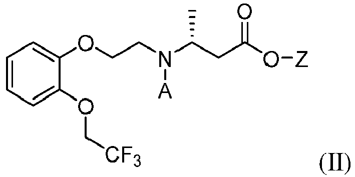 wherein **A** and **Z** are as defined above.

The compound of formula II may be prepared by different ways with reactions known by the person skilled in the art.

In particular, the compound of formula II may be prepared by the following synthetic route:

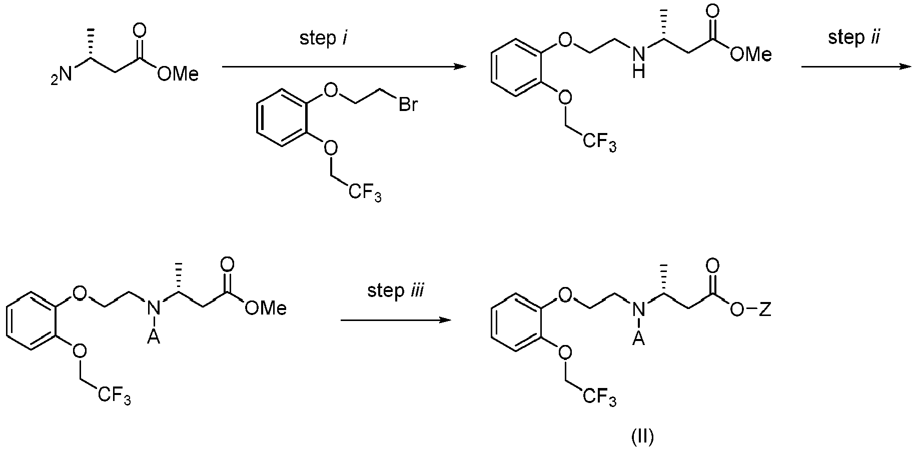

Advantageously, step *i* is performed at reflux in an organic solvent in the presence of a base.

In particular, the solvent used in step *i* is selected from aromatic non-polar solvents and polar aprotic solvents. More particularly, the solvent used in step *i* is selected from toluene, xylene, benzene, *N*,*N*-dimethylformamide, *N*,*N*-dimethylacetamide, dimethylsulfoxide, *N-*methylpyrrolidone and acetonitrile. Even more particularly, the solvent used in step *i* is toluene. Preferably, the solvent used in step *i* is selected from aromatic non-polar solvents. More preferably, the solvent used in step *i* is selected from toluene, xylene and benzene. Even more preferably, the solvent used in step i is toluene.

In particular, the base used in step *i* may be any base known by the skilled person in the art. More particularly, the base used in step *i* is selected from trimethylamine, triethylamine, diisopropylethylamine, sodium methoxide, sodium ethoxide, sodium *tert-*butoxide, sodium *tert*-pentoxide, sodium hydroxide, sodium hexamethyldisilazane, potassium methoxide, potassium ethoxide, potassium *tert*-butoxide, potassium *tert-*pentoxide, potassium hydroxide and potassium hexamethyldisilazane. Even more particularly, the base used in step i is triethylamine.

Step *ii* consists in introducing the amine protecting group **A**. In particular, **A** is a group selected from C1-C4-alkyloxycarbonyl, aryl-C1-C2-alkyloxycarbonyl, C1-C4-alkylcarbonyl, arylsulfonyl, C1-C4-alkylsulfonyl, halo-C1-C4-alkylsulfonyl, C1-C4-alkyl, C1-C4-alkenyl and aryl-C1-C4-alkyl, more particularly **A** is selected from C1-C4-alkyloxycarbonyl, aryl-C1-C2-alkyloxycarbonyl, C1-C4-alkylcarbonyl and aryl-C1-C2-alkyl, even more particularly, **A** is a group selected from *tert*-butyloxycarbonyl, benzyloxycarbonyl, acetyl and benzyl; still more particularly **A** is *tert*-butyloxycarbonyl. The amine protecting group **A** may be introduced by any method known by the skilled person in the art.

Step *iii* consists in introducing the group **Z**. In particular, **Z** is selected from H, Na, 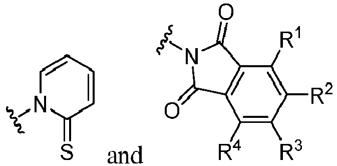 wherein **R¹**, **R²**, **R³** and **R⁴** are independently H or halo, preferably **R¹**, **R²**, **R³** and **R⁴** are independently **H** or chloro, more preferably **R¹**, **R²**, **R³** and **R⁴** are **H**. More particularly, **Z** is selected from H and 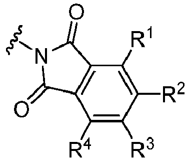 wherein **R¹**, **R²**, **R³** and **R⁴** are independently **H** or halo, preferably **R¹**, **R²**, **R³** and **R⁴** are independently H or chloro, more preferably **R¹**, **R²**, **R³** and **R⁴** are H. Still more particularly, **Z** is selected from **H** and 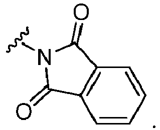 The group **Z** may be introduced by any method known by the skilled person in the art.

The compound of formula II obtained is then used in the next step.

The **step b)** of the process of the invention consists in reacting the compound of formula II with a compound of formula III: 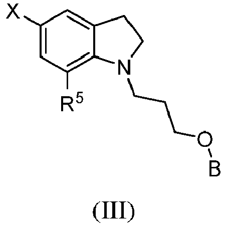 wherein **X**, **R⁵** and **B** are as defined above.

Typically, the reaction taking place during step b) between the compound of formula (II) and the compound of formula (III) is a nickel-catalyzed reductive decarboxylative cross-coupling.

Advantageously, the step b) of the process of the invention is performed in the presence of NiI₂, a ligand, a reductant and an additive.

In particular, the ligand used in step b) of the process of the invention is selected from 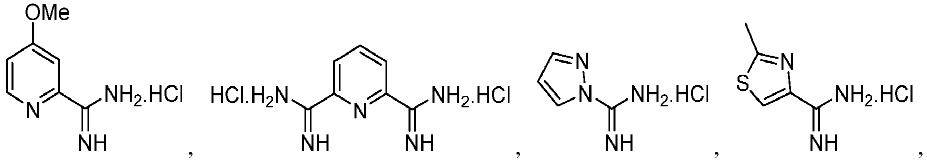 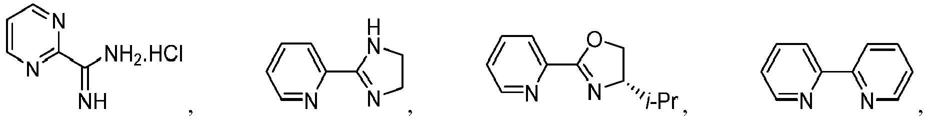 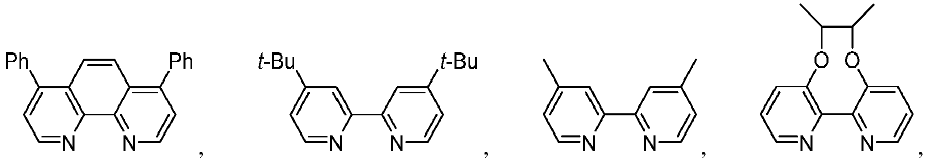 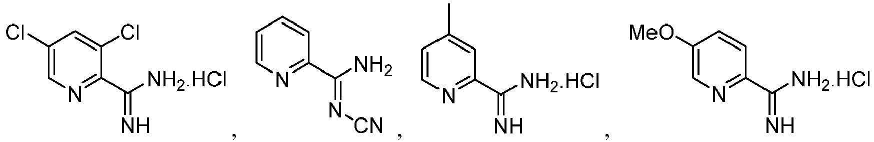 and 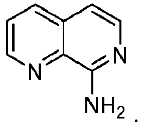 Preferably, ligand used in step b) of the process of the invention is

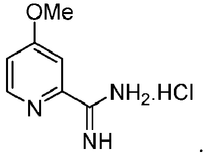

In particular, the reductant used in step b) of the process of the invention is selected from Mn, Zn and organic reductants. More particularly, the reductant used in step b) of the process of the invention is selected from Mn, Zn and tetrakis(dimethylamino)ethylene (TDAE). Preferably, the reductant used in step b) of the process of the invention is Mn.

In particular, the additive used in step b) of the process of the invention is selected from LiBr, MgCl₂, MgBr₂·Et₂O, ZnBr₂, ZnCl₂, LiCl, LiI, NaI, Bu₄NBr and NaBF₄. Preferably, the additive used in step b) of the process of the invention is LiBr.

Advantageously, the step b) of the process of the invention is performed in a polar aprotic solvent. In particular, the solvent used in step b) of the process of the invention is selected from *N*,*N*-dimethylacetamide, *N*,*N*-dimethylformamide, *N*-methylpyrrolidone, tetrahydrofuran, 2-methyl-tetrahydrofuran, 1,3-dimethyl-2-imidazolidinone and *N*,*N'-*dimethylpropyleneurea. More particularly, the solvent used in step b) of the process of the invention is *N*,*N*-dimethylacetamide.

Advantageously, the step b) of the process of the invention is performed at a temperature ranging from 20°C to 80°C, preferably from 40°C to 70°C, more preferably the step b) of the process of the invention is performed at a temperature of about 60°C.

Advantageously, the step b) of the process of the invention is performed with a molar ratio compound of formula (II)/compound of formula (III) ranging from 1:2 to 2:1. Preferably, the step b) of the process of the invention is performed with a molar ratio compound of formula (II)/compound of formula (III) of about 2:1.

In a particular embodiment, the step b) of the process of the invention is performed with a compound of formula (II) wherein **Z** is **H** or Na, particularly **Z** is H.

In this case, the presence of an activating agent is necessary in the reaction mixture during step b). The activating agent may be any activating agent known by the skilled person in the art to activate a carboxylic acid moiety. In particular, the activating agent is selected from 1-[bis(dimethylamino)methylene]-1H-1,2,3-triazolo[4,5-b]pyridinium 3-oxide hexafluorophosphate (HATU), N-hydroxyphthalimide tetramethyluronium hexafluorophosphate (PITU) and tetrachloro-N-hydroxyphthalimide tetramethyluronium hexafluorophosphate (CITU). Advantageously, the step b) of the process of the invention is performed with a molar ratio compound of formula (II) wherein **Z** is selected **H** or Na /activating agent of about 1:1.

At the end of the reaction occurring during step b), the reaction mixture may be quenched using the procedures known by the skilled person in the art, such as, for example, adding an aqueous solution of hydrochloric acid, followed by extraction with an organic solvent, preferably an ethereal solvent, more preferably a solvent selected from diethyl ether, methyl *tert*-butyl ether, 2-methyl-tetrahydrofuran, diisopropylether, and cyclopentyl methyl ether.

After the reaction of the step b) of the process of the invention is ended, the resulting product may be purified using the procedures known by the skilled person in the art, such as, for example, recrystallization or purification by column chromatography or preparative thin layer chromatography.

The **step c)** of the process of the invention consists in cleaving the groups **A** and **B** present in the product obtained after step b).

The groups **A** and **B** present in the product obtained after step b) are as defined with respect to steps a) and b) of the process of the invention.

The groups **A** and **B** may be cleaved according to any method known by the skilled person in the art. For example, the cleavage of the group **A** may be performed using reactions known by the person skilled in the art for the deprotection of a secondary amine such as, for example those reported in Greene, "Protective Groups in Organic Synthesis", John Wiley and Sons, Fourth Edition (2007). Similarly, the cleavage of the group **B** may be performed using reactions known by the person skilled in the art for the deprotection of hydroxyl such as, for example those reported in Greene, "Protective Groups in Organic Synthesis", John Wiley and Sons, Fourth Edition (2007).

The groups **A** and **B** may be cleaved successively or concomitantly.

In one embodiment, the groups **A** and **B** are cleaved concomitantly. In this case, the groups **A** and **B** may be cleaved according to any method known by the skilled person in the art that are efficient for both the deprotection of a secondary amine and the deprotection of hydroxyl, such as for example, catalytic hydrogenation in the presence of Pd/C and HCl (37% aq.).

In a particular embodiment, the step b) of the process of the invention is performed with a compound of formula (III) wherein **R⁵** is CN.

In this case, in order to obtain Silodosin, an additional step d) of hydrolysis of the CN group into C(O)NH₂ is necessary.

According to this embodiment, the process of the invention thus further comprises a step d) of hydrolysis of the CN group into C(O)NH₂.

This additional step d) of hydrolysis of the CN group may be performed by any method known by the skilled person in the art. For example, the additional step of hydrolysis of the CN group may be performed under acidic conditions, under basic conditions, in particular by treatment with a base such as potassium *tert*-butoxide, under basic oxidative conditions, in particular by treatment with a solution of hydrogen peroxide in the presence of a base such as potassium carbonate or sodium hydroxide, or under catalytic conditions.

This additional step d) of hydrolysis of the CN group may be performed before the step c), after the step c) or concomitantly with step c).

In one embodiment, the additional step d) of hydrolysis of the CN group is performed before the step c) and after the step b). In this case, the additional step of hydrolysis of the CN group may be performed under acidic conditions, under basic conditions, under basic oxidative conditions, or under catalytic conditions. Preferably, the additional step of hydrolysis of the CN group is performed under basic oxidative conditions. More preferably, the additional step of hydrolysis of the CN group is performed by treatment with a solution of hydrogen peroxide in the presence of a base. In particular, the base is selected from sodium hydroxide, potassium hydroxide, sodium carbonate and potassium carbonate. More particularly, the base is sodium hydroxide.

Silodosin or its salts or solvates may then be purified using the procedures known by the person skilled in the art, such as, for example, recrystallization or purification by column chromatography.

Another aspect of the invention concerns a compound of formula II: 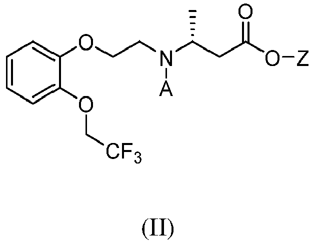 or a pharmaceutically acceptable salt thereof,
wherein
**A** is an amine protecting group, preferably **A** is a group selected from C1-C4-alkyloxycarbonyl, aryl-C1-C2-alkyloxycarbonyl, C1-C4-alkylcarbonyl, arylsulfonyl, C1-C4-alkylsulfonyl, halo-C1-C4-alkylsulfonyl, C1-C4-alkyl, C1-C4-alkenyl and aryl-C1-C2-alkyl, more preferably **A** is selected from C1-C4-alkyloxycarbonyl, aryl-C1-C2-alkyloxycarbonyl, C1-C4-alkylcarbonyl and aryl-C1-C2-alkyl, even more preferably, **A** is a group selected from *tert*-butyloxycarbonyl, benzyloxycarbonyl, acetyl and benzyl; still more preferably **A** is *tert*-butyloxycarbonyl; and
**Z** is selected from H, Na, 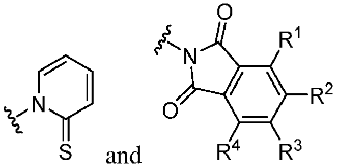 wherein **R¹, R²**, **R³** and **R⁴** are independently H or halo, preferably **R¹**, **R²**, **R³** and **R⁴** are independently H or chloro, more preferably **R¹, R²**, **R³** and **R⁴** are H; preferably, **Z** is selected from H and 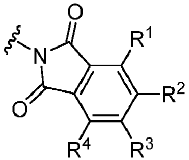 wherein **R¹**, **R²**, **R³** and **R⁴** are independently H or halo, preferably **R¹**, **R²**, **R³** and **R⁴** are independently H or chloro, more preferably **R¹, R²**, **R³** and **R⁴** are H; still more preferably,
**Z** is selected from H and

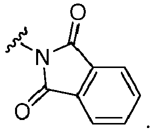

Particularly preferred compounds of the invention are those listed in Table 1 hereafter:

| Compound | Structure | Name |
|---|---|---|
| 1 | 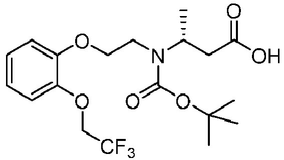 | (R)-3-((*tert*-butoxycarbonyl)(2-(2-(2,2,2-trifluoroethoxy)phenoxy)ethyl)amino)bu tanoic acid |
| 2 | 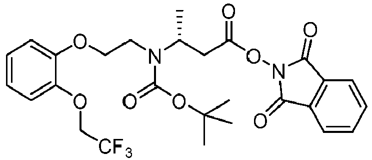 | 1,3-dioxoisoindolin-2-yl(R)-3-((*tert-*butoxycarbonyl)(2-(2-(2,2,2-trifluoroethoxy)phenoxy)ethyl)amino)bu tanoate |

Another aspect of the invention therefore concerns the use of the compound of formula II as a synthetic intermediate for the preparation of Silodosin or a pharmaceutically acceptable salt or solvate thereof.

In particular, the compound of formula II is used in the step b) of the process according to the invention consisting in the reaction of said compound of formula II with a compound of formula III.

### DEFINITIONS

The definitions and explanations below are for the terms as used throughout the entire application, including both the specification and the claims.

Unless otherwise stated any reference to compounds of the invention herein, means the compounds as such as well as their pharmaceutically acceptable salts and solvates.

When describing the process and compounds of the invention, the terms used are to be construed in accordance with the following definitions, unless indicated otherwise.

The term "amine protecting group" or "amine protective group" as used herein refers to a chemical group that is introduced into a molecule by chemical modification of a primary or a secondary amino group and, after one or more subsequent chemical reactions, which may then be removed to recover the free primary or secondary amino group. Non-limiting examples of such amine protecting group are provided in Greene, "Protective Groups in Organic Synthesis", John Wiley and Sons, Fourth Edition (2007). Preferred examples of amine protecting groups according to the invention are selected from alkyloxycarbonyl, arylalkyloxycarbonyl, alkylcarbonyl, arylsulfonyl, alkylsulfonyl, haloalkylsulfonyl, alkyl, alkenyl and arylalkyl. More preferred amine protecting groups according to the invention are selected from *tert*-butyloxycarbonyl, *tert*-butylcarbonyl, *p*-toluenesulfonyl, trifluoromethylsulfonyl, *tert-butyl,* allyl, benzyl and trityl. An even more preferred amine protecting group according to the invention is *tert*-butyloxycarbonyl.

The term "hydroxyl protecting group" or "hydroxyl protective group" or "alcohol protecting group" or "alcohol protective group" as used herein refers to a chemical group that is introduced into a molecule by chemical modification of a hydroxyl group and, after one or more subsequent chemical reactions, which may then be removed to recover the free hydroxyl group. Non-limiting examples of such hydroxyl protecting group are provided in Greene, "Protective Groups in Organic Synthesis", John Wiley and Sons, Fourth Edition (2007). Preferred examples of hydroxyl protecting groups according to the invention are selected from

The term "alkyl" by itself or as part of another substituent refers to a hydrocarbyl radical of Formula CₙH₂ₙ₊₁ wherein n is an integer greater than or equal to 1.

The term "alkenyl" by itself or as part of another substituent refers to a hydrocarbyl radical formed from an alkene by removal of one hydrogen atom of Formula CₙH₂ₙ₋₁ wherein n is an integer greater than or equal to 2.

The term "halo" or "halogen" means fluoro, chloro, bromo, or iodo. Preferred halo groups are bromo and iodo, iodo being particularly preferred.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like. Preferred haloalkyl group is trifluoromethyl.

The term "aryl" as used herein refers to a polyunsaturated, aromatic hydrocarbyl group having a single ring (*i.e.* phenyl) or multiple aromatic rings fused together (e.g. naphthyl), typically containing 5 to 12 atoms; preferably 6 to 10, wherein at least one ring is aromatic. Examples of aryl groups include but are not limited to phenyl, naphthyl, anthracyl. Preferred aryl group according to the invention is phenyl.

The term "arylalkyl" or "aralkyl" as used herein refers to a group -alkyl-aryl, wherein alkyl and aryl are as herein defined. Examples of arylalkyl groups include but are not limited to benzyl (abbreviation: Bn).

The term "alkoxy" as used herein refers to a group -O-alkyl, wherein alkyl is as herein defined. Examples of alkoxy groups include but are not limited to methoxy, ethoxy, *n-*propyloxy, isopropyloxy, *n*-butyloxy, *t*-butyloxy, *sec*-butyloxy and *n*-pentyloxy.

The term "carbonyl" or "carbonyl group" as used herein refers to a functional group composed of a carbon atom double-bonded to an oxygen atom: C=O, also defined by C(O).

The term "sulfonyl" as used herein refers to a functional group composed of a sulfur atom double-bonded to two oxygen atoms: O=S=O, also defined by SO₂.

The term "alkyloxycarbonyl" as used herein refers to a group -C(O)-alkoxy, wherein alkoxy is as herein defined. Non-limiting examples of alkyloxycarbonyl include *tert-*butyloxycarbonyl (abbreviation: Boc).

The term "arylalkyloxycarbonyl" as used herein refers to a group -C(O)-O-alkyl-aryl, wherein alkyl and aryl are as herein defined. Non-limiting examples of alkyloxycarbonyl include benzyloxycarbonyl (abbreviation: Cbz).

The term "alkylcarbonyl" as used herein refers to a group -C(O)-alkyl, wherein alkyl is as herein defined. Non-limiting examples of alkylcarbonyl include acetyl (abbreviation: Ac).

The term "arylsulfonyl" as used herein refers to a group -SO₂-aryl, wherein aryl is as herein defined.

The term "alkylsulfonyl" as used herein refers to a group -SO₂-alkyl, wherein alkyl is as herein defined.

The term "haloalkylsulfonyl" as used herein refers to a group -SO₂-haloalkyl, wherein haloalkyl is as herein defined. Non-limiting examples of haloalkylsulfonyl include trifluoromethylsulfonyl (abbreviation: Tf).

The term "alkylsulfonyloxy" as used herein refers to a group -O-alkylsulfonyl, wherein alkylsulfonyl is as herein defined.

The term "haloalkylsulfonyloxy" as used herein refers to a group -O-haloalkylsulfonyl, wherein halohaloalkyl is as herein defined.

The term "dialkylaminosulfonyloxy" as used herein refers to a group -O-SO₂-N(alkyl)₂, wherein alkyl is as herein defined.

The term "dialkylcarbamoyloxy" as used herein refers to a group -O-C(O)-N(alkyl)₂, wherein alkyl is as herein defined.

The term "reductant" or "reducing agent" or "reducer" as used herein refers to an element or compound that loses (or "donates") an electron to an electron recipient in a redox chemical reaction. Non-limiting examples of reductants include metals, such as manganese (Mn) or zinc (Zn), and organic reductants, such as tetrakis(dimethylamino)ethylene (TDAE).The term "polar aprotic solvents" as used herein refers to solvents having large dipole moments, i.e. molecules having bonds between atoms with very different electronegativities, while being unable to participate in hydrogen bonding. Non-limiting examples of polar aprotic solvents include dichloromethane, *N*-methylpyrrolidone, tetrahydrofuran, 2-methyl-tetrahydrofuran, ethyl acetate, acetone, *N*,*N*-dimethylformamide, acetonitrile, dimethylsulfoxide, *N*,*N*-dimethylacetamide, 1,3-dimethyl-2-imidazolidinone and *N*,*N'*-dimethylpropyleneurea.

The term "non-polar solvents" as used herein refers to solvents having dipole moment equal or close to zero, i.e. molecules having no polar group or molecules comprising polar groups but whose geometry causes the dipole moment to vanish. Non-limiting examples of non-polar solvents include hexane, cyclohexane, benzene, toluene, xylene, 1,4-dioxane, chloroform and diethyl ether. Non-limiting examples of aromatic non-polar solvents include benzene, toluene and xylene.

The compounds of the invention of Formula II containing a basic functional group may be in the form of pharmaceutically acceptable salts. Pharmaceutically acceptable salts of the compounds of the invention containing one or more basic functional groups include in particular the acid addition salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, cinnamate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Pharmaceutically acceptable salts of compounds of Formulae II and III and subformulae may for example be prepared as follows:
(i) reacting the compound of Formula I or any of its subformulae with the desired acid; or
(ii) converting one salt of the compound of Formula I or any of its subformulae to another by reaction with an appropriate acid or by means of a suitable ion exchange column.

All these reactions are typically carried out in solution. The salt may precipitate from solution and be collected by filtration or may be recovered by evaporation of the solvent. The degree of ionization in the salt may vary from completely ionized to almost nonionized.

The term "solvate" is used herein to describe a molecular complex comprising Rilpivirine or a pharmaceutically acceptable salt obtained by the process of the invention and one or more pharmaceutically acceptable solvent molecules. The term "hydrate" is employed when said solvent is water.

The compounds of the invention include compounds of the invention as hereinbefore defined, including all polymorphs and crystal habits thereof, prodrugs and isomers thereof (including optical, geometric and tautomeric isomers) and isotopically-labeled compounds of the invention.

In addition, although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention.

The present invention will be better understood with reference to the following examples and figures. These examples are intended to be representative of specific embodiments of the invention and are not intended as limiting the scope of the invention.

### EXAMPLES

### General experimental

Tetrahydrofuran (THF), *N*,*N*-dimethylformamide (DMF), toluene, acetonitrile (CH₃CN), and dichloromethane (CH₂Cl₂) were obtained by passing the previously degassed solvents through an activated alumina column. DMA (anhydrous, 99.5%) was purchased from Sigma-Aldrich and used without further purification.

Reagents and starting materials were purchased at the highest commercial quality from well-known chemical suppliers and used without further purification, unless otherwise stated. NiBr₂•diglyme and NiI₂ were purchased from Strem. Bipyridine ligands (2,2'-bipyridine, 4,4'-di-*tert*-butylbipyridine and 4,4'-di-OMe-bipyridine) were purchased from Sigma-Aldrich. Bathophenanthroline was purchased from Combi-Blocks.

Yields refer to chromatographically and spectroscopically (¹H NMR) homogeneous material, unless otherwise stated.

Reactions were monitored by GC/MS, LC/MS, and thin layer chromatography (TLC). TLC was performed using 0.25 mm E. Merck silica plates (60F-254), using short-wave UV light as the visualizing agent, and phosphomolybdic acid and Ce(SO₄)₂, acidic ethanolic anisaldehyde, or KMnO₄ and heat as developing agents.

NMR spectra were recorded on Bruker DRX-600, DRX-500, and AMX-400 instruments and are calibrated using residual undeuterated solvent (CHCl₃ at 7.26 ppm ¹H NMR, 77.16 ppm ¹³C NMR; acetone at 2.05 ppm ¹H NMR, 29.84 ppm ¹³C NMR; CH₃OH at 3.31 ppm ¹H NMR, 49.0 ppm ¹³C NMR; C₆H₆ at 7.16 ppm ¹ H NMR, 128.06 ppm ¹³C NMR). ¹⁹F NMR spectra were recorded using fluorobenzene (δ -113.15 ppm) as internal standard. The following abbreviations were used to explain multiplicities: s = singlet, d = doublet, t = triplet, q = quartet, m = multiplet, br = broad.

Column chromatography was performed using E. Merck silica (60, particle size 0.043-0.063 mm), and pTLC was performed on Merck silica plates (60F-254).

High-resolution mass spectra (HRMS) were recorded on an Agilent LC/MSD TOF mass spectrometer by electrospray ionization time of flight reflectron experiments.

Melting points were recorded on a Fisher-Johns 12-144 melting point apparatus and are uncorrected.

The enantiomeric excesses were determined with Waters UPC2 SFC equipped with a photodiode array detector or an Agilent Technologies 1220 Infinity II LC HPLC.

Optical rotations were recorded on a Rudolph Research Analytical Autopol III Automatic Polarimeter.

All temperatures are expressed in °C and all reactions were carried out at room temperature (RT) unless otherwise stated.

Reagents and starting materials were purchased at the highest commercial quality from well-known chemical suppliers (such as for example Sigma Aldrich, Acros Organics, Fluorochem, Eurisotop, VWR International, ABCR, TCI) and used without further purification, unless otherwise stated.

The following abbreviations are used:
DCM: dichloromethane
DIC: *N*,*N'*-Diisopropylcarbodiimide
DMA: *N*,*N*-dimethylacetamide
DMAP: 4-dimethylaminopyridine
DMF: *N*,*N*-dimethylformamide
HPLC: high performance liquid chromatography
HRMS: High-resolution mass spectra
LCMS: liquid chromatography-mass spectrometry
NBS: *N*-bromosuccinimide
NHPI: *N*-hydroxyphthalimide
NMR: Nuclear Magnetic Resonance
pTLC: preparative thin layer chromatography
TCNHPI: *N*-hydroxytetrachlorophthalimide
THF: Tetrahydrofuran
TLC: thin layer chromatography
t_{R}: retention time

### Preparation of the key intermediates of formula (II)

### Synthesis of compound 1

The synthetic route to compound **1** according to the invention is disclosed in scheme 1:

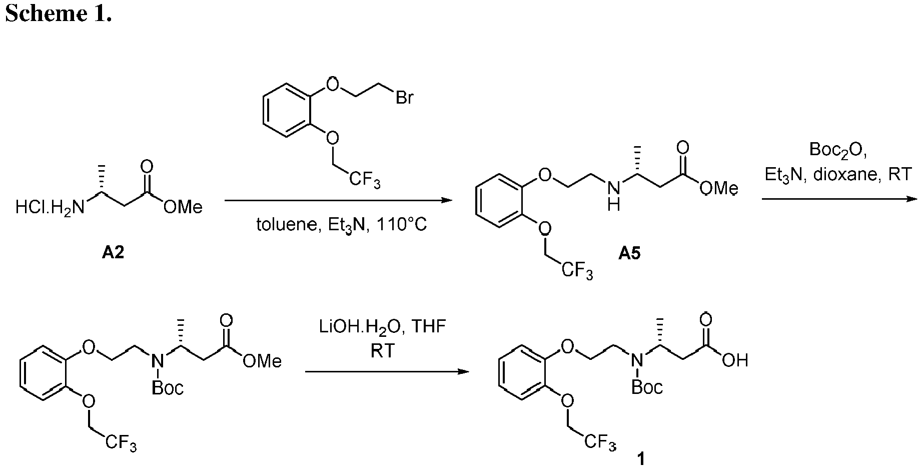

### Compound A5: Methyl (R)-3-((2-(2-(2,2,2-trifluoroethoxy)phenoxy)ethyl)amino)butanoate

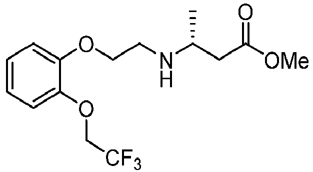

To a solution of methyl 3-aminobutanoate hydrochloride **A2** (153.6 mg, 1.0 mmol) in toluene (5.0 mL) was added 1-(2-bromoethoxy)-2-(2,2,2-trifluoroethoxy)benzene **SM1** (375 mg, 1.25 mmol) at room temperature, followed by Et₃N (202 mg, 2.0 mmol). The mixture was heated at reflux for 18 h, then cooled to room temperature, and filtered. The filtrate was concentrated under reduced pressure. Purification by pTLC (silica, 1:1 hexanes:EtOAc) afforded 90.5 mg (27%) of the title compound **A5**.
**Physical State:** yellow oil.
¹H NMR (600 MHz, CDCl₃): δ 7.04 (ddd, *J* = 8.1, 7.4, 1.6 Hz, 1H), 7.00 (dd, *J* = 8.0, 1.6 Hz, 1H), 6.94 (dd, *J* = 8.1, 1.5 Hz, 1H), 6.91 (ddd, *J* = 8.0, 7.4, 1.5 Hz, 1H), 4.44 - 4.35 (m, 2H), 4.11 (t, *J* = 5.2 Hz, 2H), 3.67 (s, 3H), 3.19 (h, *J* = 6.4 Hz, 1H), 3.08 - 2.99 (m, 2H), 2.50 (dd, *J* = 15.4, 6.7 Hz, 1H), 2.38 (dd, *J* = 15.4, 6.1 Hz, 1H), 1.89 (s, br, 1H), 1.16 (d, *J* = 6.4 Hz, 3H).
**¹³C NMR (151 MHz, CDCl₃):** δ 172.9, 150.0, 147.6, 124.4, 123.7 (q, *J*_{F-C} = 278.4 Hz), 121.6, 118.5, 114.6, 68.9, 68.6, 68.4 (q, *J*_{F-C} = 34.8 Hz), 51.6, 50.3, 46.1, 41.4, 20.5.
**¹⁹F NMR (376 MHz, CDCl₃):** δ -74.5.
**HRMS (ESI-TOF):** calculated for C₁₅H₂₁F₃NO₄ [M+H]⁺: 336.1423, found: 336.1418.
**TLC:** R*_{f}* = 0.21 (1:1 hexanes:EtOAc).

### Compound 1: (R)-3-((tert-butoxycarbonyl)(2-(2-(2,2,2-trifluoroethoxy)phenoxy)ethyl)amino)butanoic acid

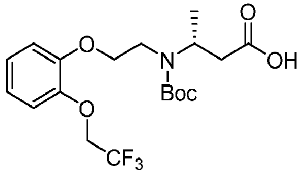

To a solution of **A5** (90.5 mg, 0.27 mmol) in dioxane (2.0 mL) was added di-*tert*-butyl dicarbonate (Boc₂O, 218.0 mg, 1.0 mmol), followed by Et₃N (101.0 mg, 1.0 mmol). The reaction mixture was stirred at room temperature for 4 h. Then the solution was concentrated under reduced pressure. Purification by pTLC (silica, 3:1 hexanes:EtOAc) afforded the crude compound, which was used for the next step without further purification.

To a solution of the above-mentioned crude material in THF (1.0 mL) was added LiOH•H₂O solution (1.0 M, 2.0 mL) at room temperature. The reaction mixture was allowed to stir until the starting material was consumed (determined by LCMS). Typical reaction times were between 4 h and 12 h. The reaction mixture was concentrated under reduced pressure. The aqueous layer was washed with ether, acidified to pH 2.0 with 4.0 M HCl solution and extracted with EtOAc (5 x 8 mL). The combined organic phase was washed with brine, dried over MgSO₄ and concentrated *in-vacuo* to afford the title compound **1** (93.7 mg, about 82% in 2 steps). The crude carboxylic acid was used directly for the next step without further purification.
**Physical State:** colorless oil.
**¹H NMR (600 MHz, CDCl₃):** δ 7.02 (td, *J* = 7.7, 1.7 Hz, 1H), 6.96 - 6.89 (m, 3H), 4.38 m, 3H) (m, 3H), 4.14 - 4.10 (m, 2H), 3.73 - m, 2(m, 2H), 2.83 (dd, *J* = 15.6, 7.6 Hz, 1H), 2.55 (dd, *J* = 15.6, 6.8 Hz, 1H), 1.46 (s, 9H), 1.32 (d, *J* = 6.9 Hz, 3H).
**¹³C NMR (151 MHz, CDCl₃):** δ 177.26, 155.5 (155.1), 149.45, 147.4 (147.2), 124.0, 123.7 (q, *J*_{F-C} = 278.5 Hz), 121.6 (121.2), 116.9, 114.2 (113.7), 80.6, 67.7 (q, *J*_{F-C} = 35.1 Hz), 51.1 (50.4), 45.8 (44.9), 40.2 (39.6), 29.8 (29.7), 28.5, 19.4 (18.8).
**¹⁹F NMR (376 MHz, CDCl)**: δ -74.3.
**HRMS (ESI-TOF):** calculated for C₁₉H₂₆F₃NO₆Na [M+Na]⁺ : 444.1610, found: 444.1602.
**TLC:** R*_{f}* = 0.38 (1:1 hexanes:EtOAc).

### General Procedure A: Synthesis of compounds of formula (II) comprising a N-hydroxyyhthalimide or N-hydroxytetrachloroyhthalimide moiety

Compounds of formula (II) comprising a *N*-hydroxyphthalimide or *N-*hydroxytetrachlorophthalimide moiety were prepared according to the procedure described in Scheme 2.

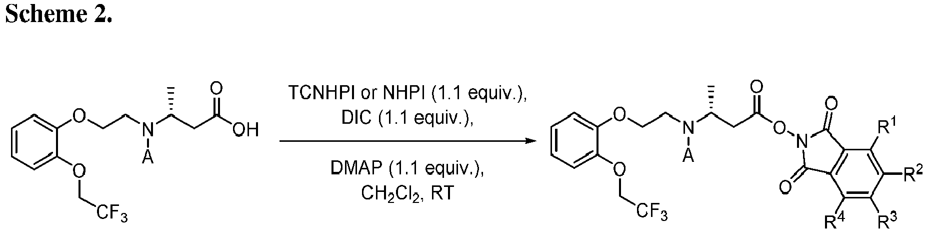

In short, a round-bottom flask or culture tube was charged with (if solid) carboxylic acid (1.0 equiv) and *N*-hydroxyphthalimide (NHPI) or *N*-hydroxytetrachlorophthalimide (TCNHPI) (1.0 - 1.1 equiv.). CH₂Cl₂ was added (0.1 - 0.2 M), and the mixture was stirred vigorously. Carboxylic acid (1.0 equiv.) was added via syringe (if liquid). DIC (1.1 equiv.) was then added dropwise via syringe, and the mixture was allowed to stir until the acid was consumed (determined by TLC). Typical reaction times were between 0.5 h and 12 h. The mixture was filtered (over Celite®, silica gel, or through a fritted funnel) and rinsed with additional CH₂Cl₂/Et₂O. The solvent was removed under reduced pressure, and purification by column chromatography afforded the desired NHPI or TCNHPI compound of formula (II).

### Compound 2: 1,3-Dioxoisoindolin-2-yl (R)-3-((tert-butoxycarbonyl)(2-(2-(2,2,2-trifluoroethoxy)phenoxy)ethyl)amino)butanoate

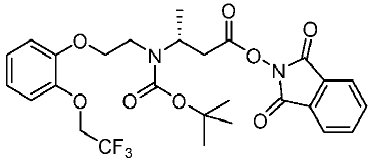

Following **General Procedure A** on 0.14 mmol scale with compound **1** and *N-*hydroxyphthalimide (NHPI). Purification by pTLC (silica, 5:1 hexanes:EtOAc) afforded 68.7 mg (82%) of the title compound **2.**
**Physical State:** yellow oil.
**¹H NMR (600 MHz, CDCl₃):** δ 7.86 (dd, *J* = 5.5, 3.1 Hz, 2H), 7.80 - 7.76 (m, 2H), 7.02 2H), 1 (m, 4H), 4.42 - 4.31 (m, 3H), 4.17 m, 3H) (m, 2H), 3.84 - 3.70 (m, 1H), 3.57 (s, br, 1H), 3.37 - 3.17 (m, 1H), 2.90 (dd, *J* = 15.5, 7.1 Hz, 1H), 1.50 - 1.43 (m, 12H).
**¹³C NMR (151 MHz, CDCl₃):** δ 167.8, 161.9, 155.2 (154.7), 149.4, 147.4 (147.2), 134.9, 129.0, 124.1, 123.9, 123.7 (q, *J*_{F-C} = 278.8 Hz), 121.5 (121.2), 116.9 (116.5), 114.1 (113.6), 80.9 (80.5), 67.5 (q, *J*_{F-C} = 35.6), 51.7 (51.5), 46.3, 37.3 (36.3), 28.6, 19.2 (18.4).
**¹⁹F NMR (376 MHz, CDCl):** δ -74.2.
**HRMS (ESI-TOF):** calculated for C₂₂H₃₀F₃N₂O₆ [M-Boc]⁺ : 467.1430, found: 467.1426.
**TLC:** R*_{f}* = 0.34 (3:1 hexanes:EtOAc).
**Chiral HPLC:** Chiralpak OD-H, 4.6 x 250 mm; 20% *i*PrOH/hexane, 1.0 mL/min, 210 nm; t_{R} (major) = 10.0 min, t_{R} (minor) = 15.9 min, 99% *ee.*

### Synthesis of compounds of formula (III)

### Synthesis of compounds of formula (III) wherein B is benzyl

The synthetic route to compounds of formula (III) wherein **B** is benzyl is disclosed in scheme 3.

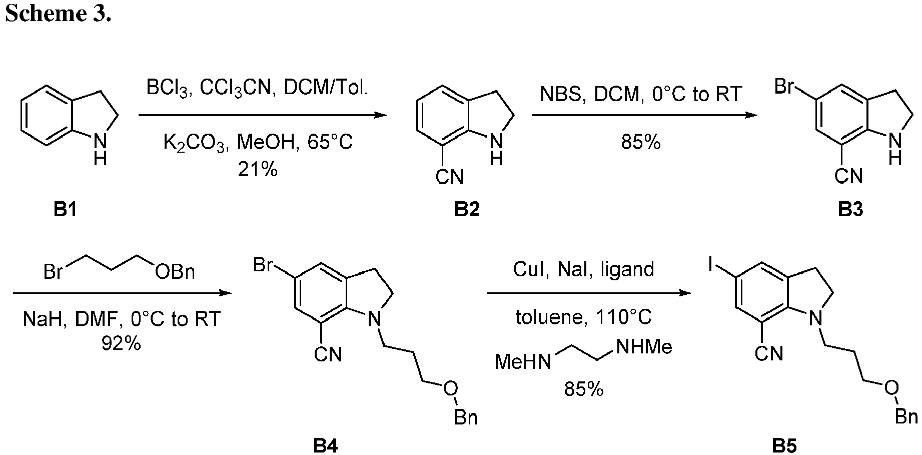

**Compound B2: Indoline-7-carbonitrile** (**B2**) (F. Calogero et al., Eur. J. Org. Chem. 2015, 27, 6011)

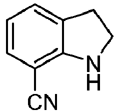

A solution of BCl₃ in toluene (1.0 M, 60 mL, 60 mmol) was put into a dried flask equipped with a stirring bar, and the solution was cooled to 0°C. A solution of indoline (6.0 g, 50 mmol) in toluene (50 mL) was added dropwise at 0°C, and the resulting suspension was heated at reflux for 1 h. The mixture was then cooled to 75°C, trichloroacetonitrile (14.4 g, 100 mmol) was added dropwise over 20 min. The resulting mixture was stirred at 85°C for 8 h. The reaction mixture was cooled to 5°C, methanol (50 mL) was added dropwise over 1 h, and then sodium methoxide (13.5 g, 250 mmol) was added, and stirring was continued at 20°C for 48 h.

After that, the reaction mixture was concentrated *in-vacuo* to remove the solvent, then added H₂O (100 mL), extracted with EtOAc (5 x 100 mL), washed with brine, dried over MgSO₄ and concentrated under reduced pressure. The crude material was purified by silica gel column chromatography (silica, 10:1 hexanes:EtOAc) to yield 1.83 g (21%) of the title compound **B2**.
**¹H NMR (600 MHz, CDCl₃):** δ 7.20 (dq, *J* = 7.2, 1.3 Hz, 1H), 7.14 (dq, *J* = 8.0, 1.0 Hz, 1H), 6.62 (dd, *J* = 8.0, 7.2 Hz, 1H), 4.44 (s, br, 1H), 3.72 (t, *J* = 8.5 Hz, 2H), 3.10 (tt, *J* = 8.4, 1.1 Hz, 2H).
**Compound B3: 5-Bromoindoline-7-carbonitrile** (F. Calogero et al., Eur. J. Org. Chem. 2015, 27, 6011)

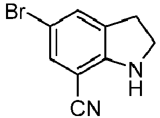

To a solution of indoline-7-carbonitrile **B2** (144.2 mg, 1.0 mmol) in CH₂Cl₂ (2.5 mL) was added NBS (196.0 mg, 1.1 mmol) at 0°C. The reaction mixture was allowed to stir at 0°C for 1 h, and then the mixture was stirred at room temperature for 2 h until the starting material was consumed (determined by LCMS). The reaction mixture was concentrated under reduced pressure. The crude material was purified by silica gel column chromatography (silica, 10:1 hexanes:EtOAc) to yield 0.1904 g (85%) of the title compound **B3.**
**Physical State:** yellowish solid.
**¹H NMR (600 MHz, CDCl₃):** δ 7.27 - 7.26 (m, 1H), 7.23 (dt, *J* = 1.9, 0.9 Hz, 1H), 4.51 (s, br, 1H), 3.73 (t, *J* = 8.6 Hz, 2H), 3.12 - 3.09 (m, 2H).
**¹³C NMR (151 MHz, CDCl₃):** δ 154.3, 132.8, 131.9, 131.1, 116.7, 108.3, 91.3, 47.2, 29.1.
**TLC:** R*_{f}* = 0.45 (3:1 hexanes:EtOAc).

### Compound B4: 1-(3-(Benzyloxy)propyl)-5-bromoindoline-7-carbonitrile

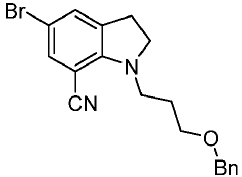

To a solution of 5-bromoindoline-7-carbonitrile **B3** (223.0 mg, 1.0 mmol) in DMF (7.0 mL) was added slowly sodium hydride (60% in mineral oil, 60 mg, 1.5 mmol) at 0°C. After the mixture was stirred at 0°C for 1 h, benzyl 3-bromopropyl ether (690 mg, 3.0 mmol) was added thereto. The resulting mixture was stirred at room temperature for 6 h. Then the reaction mixture was cooled to 0°C, quenched with H₂O, diluted with H₂O (5 mL), and extracted with ether (20 x 5 mL). The combined organic phase was washed with H₂O (10 x 2 mL), dried with MgSO₄, and concentrated under reduced pressure. The crude material was purified by silica gel column chromatography (silica, 10:1 hexanes:EtOAc) to yield 342.8 mg (92%) of the title compound **B4.**
**Physical State:** yellow oil.
**¹H NMR (600 MHz, CDCl₃):** δ 7.36 - 7.32 (m, 4H), 7.30 - 7.27 (m, 1H), 7.21 (dd, *J* = 2.0, 1.0 Hz, 1H), 7.12 (q, *J* = 1.5 Hz, 1H), 4.51 (s, 2H), 3.71 - 3.65 (m, 2H), 3.65 - 3.55 (m, 4H), 2.95 (tt, *J* = 8.5, 1.2 Hz, 2H), 1.99 - 1.95 (m, 2H).
**¹³C NMR (151 MHz, CDCl₃):** δ 152.1, 138.4, 134.9, 133.0, 131.2, 128.5, 127.82, 127.7, 118.3, 106.7, 88.7, 73.2, 67.6, 53.3, 45.0, 27.9, 27.2.
**HRMS (ESI-TOF):** calculated for C₁₉H₂₀BrN₂O [M+H]⁺: 371.0759, found: 371.0751.
**TLC:** R*_{f}* = 0.54 (3:1 hexanes:EtOAc).
**Compound B5: 1-(3-(Benzyloxy)propyl)-5-iodoindoline-7-carbonitrile** (J. Li et al., J. Med. Chem. 2017, 140, 20)

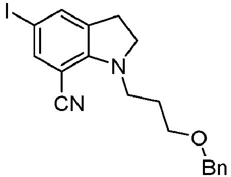

Compound **B4** (74.2 mg, 0.20 mmol), NaI (90.0 mg, 0.60 mmol), *N*, *N*-dimethylethylenediamine (7.1 mg, 0.08 mmol), CuI (7.6 mg, 0.04 mmol) and toluene (0.2 mL) were added to a tube filled with argon. The mixture was stirred at 110°C for 24 h, diluted with H₂O (2.0 mL), and extracted with EtOAc (5 mL x 3). The combined organic phase was dried over MgSO₄ and concentrated under reduced pressure. The residue was purified by pTLC (silica, 10:1 hexanes:EtOAc) to yield 71.0 mg (85%) of the title compound **B5**.
**Physical State:** yellow oil.
**¹H NMR (600 MHz, CDCl₃):** δ 7.39 - 7.37 (m, 1H), 7.35 - 7.31 (m, 4H), 7.30 - 7.26 (m, 2H), 4.50 (s, 2H), 3.69 - 3.67 (m, 2H), 3.62 - 3.58 (m, 4H), 2.94 (ddt, *J* = 9.6, 8.5, 1.1 Hz, 2H), 1.99 - 1.94 (m, 2H).
**¹³C NMR (151 MHz, CDCl₃):** δ 152.5, 139.1, 138.4, 136.4, 135.1, 128.5, 127.9, 127.8, 118.2, 89.9, 74.6, 73.3, 67.7, 53.2, 45.0, 28.0, 27.1.
**HRMS (ESI-TOF):** calculated for C₁₉H₂₀IN₂O [M+H]⁺: 419.0620, found: 419.0614.
**TLC:** R*_{f}* = 0.53 (3:1 hexanes:EtOAc).

### General Procedure B: Nickel-catalyzed Reductive Decarboxylative Cross-Coupling

The Nickel-catalyzed Reductive Decarboxylative Cross-Coupling of redox-active esters was conducted according to a procedure previously reported (K. M. M. Huihui et al., J. Am. Chem. Soc. 2016, 138, 5016).

### Part I. Preparation of Nickel catalyst (NiI₂/Ligand, 0.2 M in DMA)

A culture tube was charged with NiI₂ (124.0 mg, 0.4 mmol) and 2-amidinopyridine hydrochloride (64.0 mg, 0.4 mmol). The tube was then evacuated and backfilled with argon from a balloon 3 times. DMA (2.0 mL) was added and the resulting mixture was stirred at 60°C for 1 hour to give a homogeneous green solution, which could be used for several days without appreciable deterioration.

### Part II. Ni-catalyzed Reductive Decarboxylative Cross-Coupling

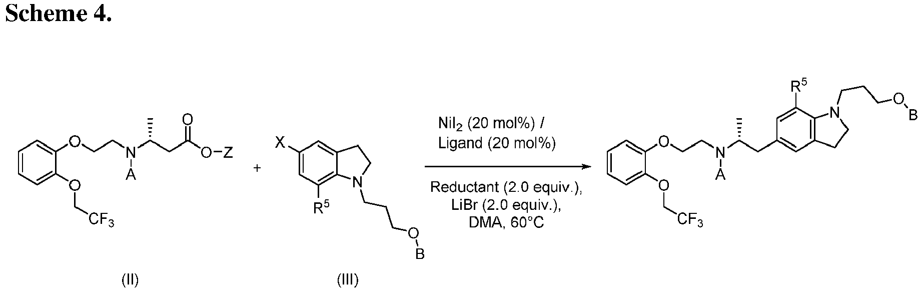

In a 10 mL screwed-capped vial, LiBr (8.7 mg, 0.1 mmol) was weighed. The vial was sealed, heated under vaccum with the help of a heat gun and back-filled with Ar. The compound of formula (II) (0.2 mmol), the compound of formula (III) (37.1 mg, 0.1 mmol for aryl bromide or 41.8 mg, 0.1 mmol for aryl iodide) and reductant (13.0 mg, 0.2 mmol for Zn powder or 11.0 mg, 0.2 mmol for Mn powder) were weighed. The vial was then sealed and evacuated and back-filled with Ar. Then, a solution of Nil/ligand (0.2 M in DMF, 0.1 mL, 20 mol%) was added and the resulting mixture was stirred at room temperature for 1 min, and then placed in an oil bath preheated at 60°C for 12 hours under Ar. The mixture was diluted with EtOAc (5.0 mL) and then quenched with 1.0 M HCl (aq). Et₂O (20 mL) was added, and the organic layer was washed with H₂O and brine, dried over anhydrous Na₂SO₄, and concentrated under reduced pressure. The crude material was purified by silica gel column chromatography or preparative TLC (pTLC).

**Compound P3: *tert*-Butyl (*R*)-(1-(1-(3-(benzyloxy)propyl)-7-cyanoindolin-5-yl)propan-2-yl)(2-(2-(2,2,2-trifluoroethoxy)phenoxy)ethyl)carbamate** (F. Calogero et al., Eur. J. Org. Chem. 2015, 27, 6011; P.-C. Yan et al., Tetrahedron Lett. 2013, 54, 1449)

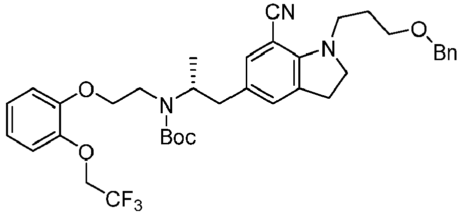

Following **General Procedure B** on 0.04 mmol scale with compounds **2** and **B5** as starting materials in the presence of Mn as reductant and 4-methoxypicolinimidamide as ligand. Purification by pTLC (silica, 10:1:1 hexanes:EtOAc:Et₂O) afforded 10.7 mg (40%) of the title compound **P3**.
**Physical State:** yellowish oil.
**¹H NMR (600 MHz, CDCl₃):** δ 7.35 30 MHz (m, 4H), 7.30 - 7.27 (m, 1H), 7.04 - 6.88 (m, 6H), 4.51 (s, 2H), 4.36 (q, *J* = 8.4 Hz, 2H), 4.14 - 3.88 (m, 3H), 3.66 - 3.61 (m, 4H), 3.55 - 3.37 (m, 4H), 2.90 - 2.71 (m, 3H), 2.54 - 2.51 (m, 1H), 1.98 m, 1H) (m, 2H), 1.43 (s, 9H), 1.23 (s, br, 3H).
**¹³C NMR (151 MHz, CDCl₃):** δ 155.7, 155.1, 151.8, 149.6 (149.5), 147.5 (147.2), 138.5, 132.8, 131.5, 129.6 (129.6), 128.5, 127.9, 127.7, 124.2 (124.1), 123.7(q, *J*_{F-C} = 278.0 Hz), 121.6 (121.2), 119.7, 117.3, 114.3 (113.8), 87.7 (87.5), 80.0, 73.3, 67.9 (q, *J*_{F-C} = 35.1 Hz), 67.9 (67.5), 55.1 (54.3), 53.4, 45.4, 44.1(43.9), 40.3 (39.9), 29.9, 28.6, 28.0, 27.5, 19.2, 18.4.
**¹⁹F NMR (376 MHz, CDCl):** δ -74.2.
**HRMS (ESI-TOF):** calculated for C₃₇H₄₅F₃N₃O₅ [M+H]⁺: 668.3311, found: 668.3320.
**TLC:** R*_{f}* = 0.47 (3:1 hexanes:EtOAc).
**Chiral HPLC:** Chiralpak IC, 4.6 x 250 mm; 30% *i*PrOH/hexane, 1.0 mL/min, 210 nm; t_{R} (major) = 6.3 min, t_{R} (minor) = 6.9 min, 99% *ee.*

### Hydrolysis of the nitrile group and deprotection of the secondary amine and the hydroxyl group

The hydrolysis of the nitrile group and the deprotection of the secondary amine and the hydroxyl group of compound **P3** may be conducted according to a procedure previously reported (F. Calogero et al., Eur. J. Org. Chem. 2015, 27, 6011). According to this procedure, hydrolysis of the nitrile group of compound P3 was achieved using H₂O₂ (50%) and NaOH in DMSO. Catalytic hydrogenation in the presence of HCl (37% aq.) of the resulting compound allowed the double deprotection of both *tert*-butyl carbamate and benzyl groups in order to obtain silodosin after purification.

## Claims

1. Process for the preparation of Silodosin or a pharmaceutically acceptable salt or solvate thereof comprising the following steps in the following order:
a) preparing a compound of formula II: 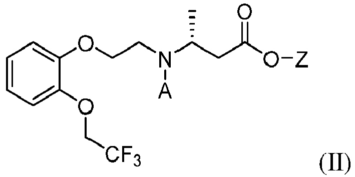 wherein
**A** is an amine protecting group, and
**Z** is selected from H, Na, 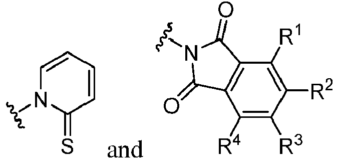 wherein **R¹**, **R²**, **R³** and **R⁴** are independently H or halo;
b) reacting said compound of formula II with a with a compound of formula III: 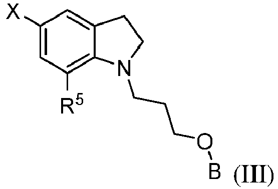 wherein
**X** is selected from halogen, C1-C6-alkoxy, C1-C6-alkylsulfonyloxy, halo-C1-C6-alkylsulfonyloxy, di-C1-C6-alkylaminosulfonyloxy and di-C1-C6-alkylcarbamoyloxy,
**R⁵** is CN or C(O)NH₂, and
**B** is an hydroxyl protecting group;
c) cleaving the groups **A** and **B**.

2. Process according to claim 1, wherein **A** is a group selected from C1-C4-alkyloxycarbonyl, aryl-C1-C2-alkyloxycarbonyl, C1-C4-alkylcarbonyl, arylsulfonyl, C1-C4-alkylsulfonyl, halo-C1-C4-alkylsulfonyl, C1-C4-alkyl, alkenyl and aryl-C1-C2-alkyl.

3. Process according to claim 1, wherein **B** is a group selected from aryl-C1-C6-alkyl, arylcarbonyl, C1-C6-alkylcarbonyl, C1-C6-alkyl, C1-C6-alkenyl, C1-C6-alkyldi-C1-C4-alkylsilyl, C1-C6-alkyldiarylsilyl and tetrahydropyranyl.

4. Process according to any of claims 1 to 3, wherein the step b) of the process of the invention is performed in the presence of NiI₂, a ligand, a reductant and an additive.

5. Process according to claim 4, wherein the ligand is selected from

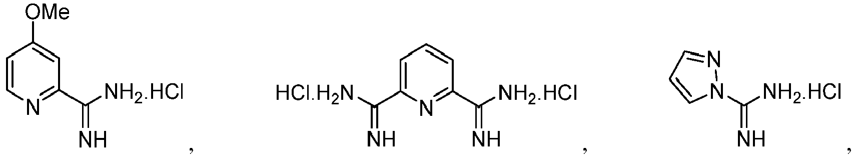

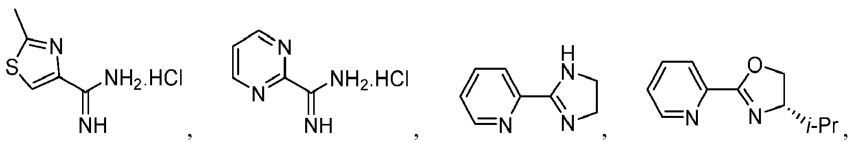

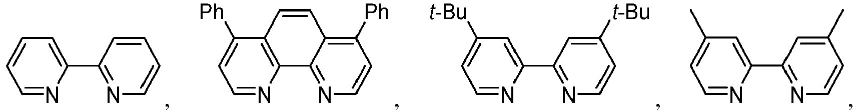

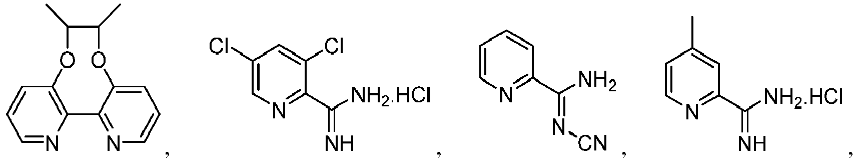

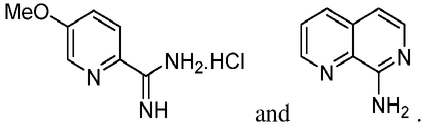

6. Process according to claim 4, wherein the reductant is selected from Mn, Zn and organic reductants.

7. Process according to claim 4, wherein the additive is selected from LiBr, MgCl₂, MgBr₂·Et₂O, ZnBr₂, ZnCl₂, LiCl, LiI, NaI, Bu₄NBr and NaBF₄.

8. Process according to any of claims 1 to 7, wherein the step b) is performed in a polar aprotic solvent.

9. Process according to any of claims 1 to 7, wherein the step b) is performed at a temperature ranging from 20°C to 80°C.

10. Process according to any of claims 1 to 6, wherein the step b) is performed with a molar ratio compound of formula (II)/compound of formula (III) ranging from 1:2 to 2:1.

11. Process according to any of claims 1 to 7, wherein step b) is performed with a compound of formula (III) wherein **R⁵** is CN.

12. Process according to claim 11, further comprising a step d) of hydrolysis of the CN group into C(O)NH₂.

13. Compound of formula II: 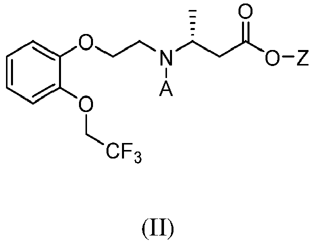 or a pharmaceutically acceptable salt thereof,
wherein
**A** is an amine protecting group, and
**Z** is selected from H, Na, 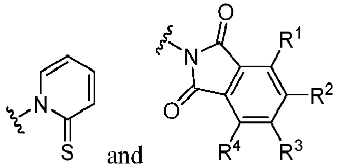 wherein **R¹**, **R²**, **R³** and **R⁴** are independently H or halo.

14. The compound according to claim 13, wherein **A** is selected from C1-C4-alkyloxycarbonyl, aryl-C1-C2-alkyloxycarbonyl, C1-C4-alkylcarbonyl and aryl-C1-C2-alkyl.

15. The compound according to claim 13, selected from the group consisting of:
1,3-dioxoisoindolin-2-yl (R)-3-((tert-butoxycarbonyl)(2-(2-(2,2,2-trifluoroethoxy)phenoxy)ethyl)amino)butanoate; and
1,3-dioxoisoindolin-2-yl (R)-3-(((benzyloxy)carbonyl)(2-(2-(2,2,2-trifluoroethoxy)phenoxy)ethyl)amino)butanoate.

16. Use of the compound according to any one of claims 13 to 15 as a synthetic intermediate for the preparation of Silodosin or a pharmaceutically acceptable salt or solvate thereof.
